Europäisches Patentamt

**European Patent Office**

Office européen des brevets

(19)

(11) Numéro de publication: **0 013 851**
**B1**

(12) FASCICULE DE BREVET EUROPEEN

(45) Date de publication du fascicule du brevet: **23.06.82**

(51) Int. Cl.³: **C 12 P 21/00, A 61 K 35/74**

(21) Numéro de dépôt: **79401029.8**

(22) Date de dépôt: **18.12.79**

(54) Protéoglycanes bactériens purifiés, procédé pour leur préparation et vaccin les contenant.

(30) Priorité: **19.12.78 FR 7835649**

(43) Date de publication de la demande:
**06.08.80 Bulletin 80/16**

(45) Mention de la délivrance du brevet:
**23.06.82 Bulletin 82/25**

(84) Etats contractants désignés:
**AT BE CH DE GB IT LU NL SE**

(56) Documents cités:
FR - A - 1 300 714
FR - A - 2 253 499
FR - A - 2 258 863
FR - A - 2 261 755
FR - A - 2 273 067
FR - A - 2 316 964
FR - A - 2 351 664
FR - A - 2 361 910
US - A - 4 096 073

(73) Titulaire: **PIERRE FABRE S.A.**
**125, rue de la Faisanderie**
**F-75116 Paris (FR)**

(72) Inventeur: **Dussourd d'Hinterland, Lucien**
**Domaine de Plombières Avenue de Lautrec**
**F-81100 Castres (FR)**
Inventeur: **Normier, Gérard**
**23, rue Francis Poullenc**
**F-81100 Castres (FR)**
Inventeur: **Pinel, Anne-Marie**
**22, rue des Capucins**
**F-81100 Castres (FR)**
Inventeur: **Durand, Jacques**
**6, rue du Béarn**
**F-81100 Castres (FR)**

(74) Mandataire: **Corre, Jacques Denis Paul et al,**
**Cabinet Regimbeau 26, Avenue Kléber**
**F-75116 Paris (FR)**

Courier Press, Leamington Spa, England.

**0 013 851**

Protéoglycanes bactériens purifiés, procédé pour leur préparation et vaccin les contenant

La présente invention concerne des protéoglycanes membranaires bactériens purifiés et détoxifiés ainsi qu'un procédé pour leur préparation et leur application comme adjuvant d'immunité dans les vaccins.

Il faut rappeler que les vaccins sont, en général, constitués par un ou plusieurs éléments immunogènes, c'est-à-dire susceptibles de faire apparaître une réaction immunitaire.

Dans certains cas, toutefois, les éléments immunogènes sont incapables de développer seuls leur pouvoir immunogénique ou bien ne le développent qu'à un degré extrêmement faible, il faut alors avoir recours à des produits appelés "adjuvants d'immunité".

Les adjuvants d'immunité sont classiquement des produits qui ont une activité immunostimulante sans être immunogènes par eux-mêmes; on peut citer par exemple l'adjuvant de Freund incomplet (IFA) qui est composé de 85% de paraffine et de 15% de naphtalène.

La présente invention concerne donc des adjuvants d'immunité utiles dans les vaccins et plus particulièrement dans les vaccins dits ribosomaux ou à base d'ARN. Ce type de vaccins ribosomaux a été décrit notamment dans le brevet français n° 2 253 499.

Il s'agit de vaccins contenant à titre de composé immunogène, soit des ribosomes bactériens, soit des ARN bactériens provenant des bactéries pathogènes contre lesquelles on désire obtenir une immunité. Toutefois, comme cela est décrit dans le brevet précédent, ces composés immunogènes ne peuvent agir complètement qu'en présence d'adjuvants d'immunité constitués par les protéoglycanes membranaires extraits de membranes de bactéries.

Toutefois, les procédés connus actuellement, tels que le procédé décrit dans le brevet français n° 2 253 499, pour la préparation de ces protéoglycanes, ne permettent pas une purification et une détoxification complètes de ces protéoglycanes, ce qui peut conduire dans certains cas à des réactions vaccinales ou des réactions pyrogéniques qu'il est intéressant de pouvoir éviter. En outre, les protéoglycanes obtenus par la mise en oeuvre du procédé selon le brevet français n° 2 253 499 ne présentent qu'une solubilité très limitée dans l'eau.

En particulier, la présente invention concerne un procédé permettant de purifier les protéoglycanes afin d'obtenir des protéoglycanes membranaires bactériens solubles dans l'eau ce qui constitue un avantage considérable car les protéoglycanes ainsi obtenus peuvent être stérilisés par filtration et donc sans avoir recours à la chaleur qui jusqu'alors pouvait dénaturer leurs propriétés. C'est pourquoi, dans le cadre de la présente invention, il faudra entendre par protéoglycanes purifiés des protéoglycanes solubles dans l'eau.

Le brevet français n° 2 261 755 décrit un procédé permettant la préparation d'extraits biologiques contenant des glycoprotéines, des mucopolysaccharides et des substances diverses à partir d'échantillons biologiques par un traitement alcalin et un chauffage à 110°C ou une mise en contact intime avec de l'oxyde d'éthylène. Les produits obtenus dans la mise en oeuvre de ce procédé sont en grande partie soit dégradés par le chauffage, soit réagissent avec l'oxyde d'éthylène pour donner des produits de structures diverses, ce qui conduit à des produits présentant des propriétés non reproductibles et à des composés inutilisables dans le cadre des vaccins.

C'est pourquoi la présente invention concerne un procédé destiné à purifier et à détoxifier les protéoglycanes membranaires bactériens, des protéoglycanes membranaires purifiés et leur application en tant qu'adjuvant d'immunité dans les vaccins.

La présente invention concerne un procédé de préparation des protéoglycanes membranaires bactériens solubles purifiés, caractérisé en ce qu'il comporte au moins une étape de traitement des protéoglycanes bruts en milieu aqueux par une base ou un hypobromite suivie par l'élimination de l'excès de réactif et du résidu insoluble, les protéoglycanes purifiés se trouvant en solution aqueuse.

Dans un mode de mise en oeuvre du procédé selon la présente invention, les protéoglycanes bruts sont traités par un hydroxyde alcalin, en particulier la soude, dans l'eau à une molarité comprise entre 0,05 et 2 M, de préférence 0,1 M, le traitement étant poursuivi quelques heures par exemple 24 heures à température voisine de l'ambiante, par exemple 25°C, et de préférence sous agitation. Ce traitement a pour effet d'hydrolyser de façon ménagée les protéoglycanes afin d'en extraire la fraction soluble.

Dans ce mode de mise en oeuvre, afin d'éliminer l'excès de réactif, l'hydrolysat est neutralisé par un acide tel que l'acide chlorhydrique et le sel ainsi formé est éliminé par exemple par dialyse contre de l'eau distillée. Il reste alors un résidu insoluble que l'on peut éliminer par précipitation, en particulier par centrifugation, par exemple 45 minutes à 30 000 g et 4°C. Le surnageant constitue les protéoglycanes solubles purifiés.

Dans un second mode de mise en oeuvre du procédé selon l'invention, les protéoglycanes bruts dans l'eau sont traités par un hypobromite alcalin pendant une durée de l'ordre de quelques dizaines de minutes, de préférence à la température ambiante et sous agitation, puis l'hypobromite en excès peut être éliminé en particulier par dialyse de 24 heures contre de l'eau courante puis contre de l'eau distillée. Le résidu insoluble peut être éliminé comme indiqué précédemment.

Le procédé selon la présente invention comporte, en outre, de préférence une étape d'élimination des lipides A par hydrolyse acide en milieu aqueux, de préférence à température comprise entre 70 et 100°C en présence d'environ 1% d'acide acétique glacial pendant une durée de l'ordre de 90 minutes

2

**0 013 851**

et élimination du précipité formé.

La présente invention concerne également à titre d'adjuvant d'immunité dans les vaccins, des protéoglycanes membranaires bactériens solubles dans l'eau, purifiés, obtenus par la mise en oeuvre du procédé précédent, caractérisés en ce qu'ils ont la composition suivante en poids:

| | |
|---|---|
| Hexoses | 24—42% |
| Protéines | 31—53% |
| Lipides | 12—18% |
| Hexosamines | 2—6% |
| ARN, moins de | 0,5% |
| ADN moins de | 0,2% |
| LPS moins de | 0,001% |

Parmi ces protéoglycanes, il faut citer en particulier les protéoglycanes ayant la composition suivante:

| | |
|---|---|
| Hexoses | $37\pm5\%$ |
| Protéines | $36,5\pm5\%$ |
| Lipides | $15\pm3\%$ |
| Hexosamines | $5\pm2\%$ |
| ARN | $<0,5\%$ |
| ADN | $\ll0,2\%$ |
| LPS | $<0,001\%$ |

qui peuvent être obtenus notamment par la mise en oeuvre du procédé précédent dans laquelle on utilise un hydroxyde alcalin.

Il faut également mentionner parmi les protéoglycanes de l'invention les protéoglycanes ayant la composition suivante:

| | |
|---|---|
| Hexoses | $29\pm5\%$ |
| Protéines | $48\pm5\%$ |
| Lipides | $15\pm3\%$ |
| Hexosamines | $4\pm2\%$ |
| ARN | $<0,5\%$ |
| ADN | $\ll0,2\%$ |
| LPS | $<0,001\%$ |

qui peuvent être obtenus notamment par la mise en oeuvre du procédé précédent dans laquelle on utilise un hypobromite.

Afin de caractériser les différentes fractions, on peut utiliser les méthodes analytiques suivantes:
*Hexoses:* par la réaction à l'anthrone selon SCOTT (T.A.), MELVIN (E.H.) 1953, Analyt. Chem. 25, 1956.
*Protéines:* par la réaction du Biuret selon GORNALL (A.G.), BARDAXILL (C.A.) DAVID (V.V.), 1949, J. Biol. Chem., 177,751.
*Lipides:* par une méthode gravimétrique basée sur une extraction globale par des solvants organiques.
*Hexosamines:* par leur réaction avec la p. diméthylaminobenzaldéhyde d'après MORGAN (W.T.S.), RONDEL (C.J.M.), 1955, Biochem. J. 61
*ARN:* (acide ribonucléique): par spectrophotométrie u.v. directe à 260 nm et par la réaction à l'orcinol

3

**0013851**

selon LUSENA (C.V.), 1951, Canad. J. Biochem., 29, 107—108

*ADN:* (acide désoxyribonucléique) par la réaction colorée à la diphénylamine d'après BURTON (K), 1956, Biochem. J. 62,315

*LPS:* (Lipopolysaccharides), par la réaction colorée à la carbocyanine décrite par JANDA (J.), WORK (E.), 1971, FEBS Letters, vol. 16, n° 4 p. 343—345

*Eau résiduelle:* par la réaction de Karl FISCHER.

En général, les protéoglycanes membranaires bruts peuvent être préparés par un quelconque procédé connu, en particulier par le procédé décrit dans le brevet mentionné ou par centrifugation fractionnée à partir d'une biomasse bactérienne broyée afin de sédimenter successivement les débris cellulaires sous une accélération comprise de préférence entre 7 000 et 8 000 g pendant quelques minutes, puis les membranes sous une accélération de 20 000 à 40 000 g pendant quelques dizaines de minutes, puis séparation des protéoglycanes des membranes par centrifugation fractionnée en solution saline et dans l'eau.

Le schéma suivant donne un exemple d'obtention de protéoglycanes purifiés à partir d'une biomasse bactérienne.

**0 013 851**

Représentation schématique de la préparation des protéoglycanes

Biomasse

↓

Broyage

↓

Centrifugation 7.500 g — 10 mn

↓

Centrifugation 30.000 g — 45 mn

↓

Reprise NaCl 0,15 M

↓

Centrifugation 7.500 g — 10 mn

↓

Centrifugation 30.000 g — 45 mn

↓

Reprise $H_2O$

↓

Centrifugation 7.500 g — 10 mn

↓

Centrifugation 30.000 g — 45 mn

↓

Reprise $H_2O$

↓

Centrifugation 7.500 g — 10 mn

↓

Surnageant : protéoglycanes bruts

| Hydrolyse par NaOH 0,1 N | Hydrolyse par BrONa |
|---|---|
| ↓ | ↓ |
| Neutralisation | Dialyse |
| ↓ | ↓ |
| Dialyse | Centrifugation 30.000 g |
| ↓ | |
| Centrifugation 30.000 g | |
| ↓ | ↓ |
| Surnageant | Surnageant |

Protéoglycanes solubles

↓

Hydrolyse par $CH_3COOH$ 1%

↓

Centrifugation 30.000 g

↓

Dialyse

↓

Stérilisation

→ Protéoglycanes purifiés, détoxifiés

**0013851**

Les protéoglycanes membranaires selon la présente invention qui sont particulièrement intéressants sont extraits des membranes des bactéries gram négatif, en particulier des genres Klebsiella, Serratia et Escherichia et tout particulièrement de

Klebsiella pneumoniae

Serratia marcescens

Escherichia coli.

La présente invention concerne également les vaccins contenant des protéoglycanes membranaires décrits précédemment et en particulier les vaccins contenant à titre de fraction immunogénique les ribosomes ou les ARN extraits de bactéries pathogènes contre lesquelles une immunité est désirée.

Les ARN et les ribosomes utilisables dans les vaccins selon l'invention peuvent être préparés par des procédés connus en particulier les procédés décrits dans les brevets français n° 2 253 499, 2 305 990 et 2 360 314.

Dans un mode de réalisation de cet aspect de l'invention, il est prévu que le rapport en poids entre la fraction immunogénique et les protéoglycanes de l'invention soit compris entre 1,4 et 1,6 et de préférence 1,5.

On peut citer tout particulièrement les vaccins suivants:

1) *Vaccins Broncho-ORL*

    a) *Association Ribosomes-protéoglycanes*

| | |
|---|---|
| —ribosomes de Klebsiella pneumoniae | 3,5 μg |
| —ribosomes de Streptococcus pneumoniae | 3,0 μg |
| —ribosomes de Streptococcus pyogènes $A_{12}$ | 3,0 μg |
| —ribosomes d'Hemophilus influenzae | 0,5 μg |
| —Protéoglycanes de Klebsiella pneumoniae | 15,0 μg |

    b) *Association ARN-ribosomal-protéoglycanes*

| | |
|---|---|
| —ARN ribosomal de Klebsiella pneumoniae | 2,45 μg |
| —ARN ribosomal de Streptococcus pneumoniae | 2,10 μg |
| —ARN ribosomal de Streptococcus pyogènes $A_{12}$ | 2,10 μg |
| —ARN ribosomal d'Hemophilus influenzae | 0,35 μg |
| —Protéoglycanes de Klebsiella pneumoniae | 10,50 μg |

2) *Vaccins anti-pyorrhéiques*

    a) *Association ribosomes-protéoglycanes*

| | |
|---|---|
| —ribosomes de Rothia dentocariosus | 1,2 μg |
| —ribosomes d'Actinomyces viscosus | 1,2 μg |
| —ribosomes de Streptococcus mutans | 0,8 μg |
| —ribosomes de Streptococcus salivarius | 2,0 μg |
| —ribosomes de Lactobacillus casei | 1,2 μg |
| —Protéoglycanes de Klebsiella pneumoniae | 9,6 μg |

    b) *Association ARN-ribosomal Protéoglycanes*

| | |
|---|---|
| —ARN ribosomal de Rothia dentocariosus | 0,84 μg |
| —ARN ribosomal d'Actinomyces viscosus | 0,84 μg |

**0 013 851**

—ARN ribosomal de Streptococcus mutans .......... 0,56 $\mu$g

—ARN ribosomal de Streptococcus salivarius .......... 1,40 $\mu$g

—ARN ribosomal de Lactobacillus casei .......... 0,84 $\mu$g

—Protéoglycanes de Klebsiella pneumoniae .......... 6,72 $\mu$g

3) *Vaccin dermatologique, anti-acnéique*

a) *Association ribosomes—Protéoglycanes*

—ribosomes de Corynebacterium acnes .......... 2 $\mu$g

—ribosomes de Corynebacterium parvum .......... 2 $\mu$g

—ribosomes de Streptococcus pyogènes .......... 2 $\mu$g

—ribosomes de Staphylococcus epidermidis .......... 2 $\mu$g

—Protéoglycanes de Serratia marcescens .......... 12 $\mu$g

b) *Association ARN ribosomal—Protéoglycanes*

—ARN ribosomal de Corynebacterium acnes .......... 1,4 $\mu$g

—ARN ribosomal de Corynebacterium parvum .......... 1,4 $\mu$g

—ARN ribosomal de Streptococcus pyogènes .......... 1,4 $\mu$g

—ARN ribosomal de Staphylococcus epidermidis .......... 1,4 $\mu$g

—Protéoglycanes de Serratia marcescens .......... 8,4 $\mu$g

4) *Vaccin gynécologique*

a) *Association ribosomes-protéoglycanes*

—ribosomes d'Escherichia coli .......... 2 $\mu$g

—ribosomes de Streptococcus faecalis D .......... 2 $\mu$g

—ribosomes de Streptococcus H .......... 2 $\mu$g

—ribosomes de Staphylococcus epidermidis .......... 2 $\mu$g

—ribosomes de Candida albicans .......... 2 $\mu$g

—Protéoglycanes d'Escherichia coli .......... 15 $\mu$g

b) *Association ARN ribosomal Protéoglycanes*

—ARN ribosomal d'Escherichia coli .......... 1,4 $\mu$g

—ARN ribosomal de Streptococcus faecalis D .......... 1,4 $\mu$g

—ARN ribosomal de Streptococcus H .......... 1,4 $\mu$g

—ARN ribosomal de Staphylococcus epidermidis .......... 1,4 $\mu$g

—ARN ribosomal de Candida albicans .......... 1,4 $\mu$g

—Protéoglycanes d'Escherichia coli .......... 10,5$\mu$g

7

**0013851**

5) *Vaccin intestinal, anti-typhique*

    a) *Association Ribosomes-Protéoglycanes*

| | |
|---|---|
| —ribosomes de Bacterium coli | 2 μg |
| —ribosomes de Salmonella paratyphi A | 2 μg |
| —ribosomes de Salmonella paratyphi B | 2 μg |
| —ribosomes de Shigella dysenteria | 2 μg |
| —ribosomes d'Enterococcus | 2 μg |
| —Protéoglycanes de Serratia marcescens | 15 μg |

    b) *Association ARN ribosomal—Protéoglycanes*

| | |
|---|---|
| —ARN ribosomal de Bacterium coli | 1,4 μg |
| —ARN ribosomal de Salmonella paratyphi A | 1,4 μg |
| —ARN ribosomal de Salmonella paratyphi B | 1,4 μg |
| —ARN ribosomal de Shigella dysenteriae | 1,4 μg |
| —ARN ribosomal d'Enterococcus | 1,4 μg |
| —Protéoglycanes de Serratia marcescens | 10,5 μg |

Exemple 1
*Préparation de protéoglycanesmembraniares bruts K. pneumoniae*

La souche de K. pneumoniae est cultivée sur un milieu contenant par litre:

| | |
|---|---|
| Extrait de viande | 5 g |
| Extrait de levure | 5 g |
| Saccharose | 2 g |
| Phosphate disodique | 2,5 g |

Les cellules sont ensuite séparées du milieu de culture par centrifugation puis lavées avec une solution saline (NaCl) et séchées et éventuellement conservées à −20°C.

La biomasse obtenue par fermentation est concentrée et lavée par centrifugation continue. Le concentrat bactérien ainsi obtenu est dispersé dans du sérum physiologique pour avoir une concentration finale en cellules sèches de 50 g par litre (mesure par spectrophotométrie).

La suspension bactérienne est soumise à un broyage cellulaire par passage dans un homogénéiseur APV-Manton Gaulin équipé de clapets spéciaux de désintégration. Pendant cette opération, la température de la suspension est maintenue au-dessous de 10°C.

Les protéoglycanes bruts sont isolés de ce broyat cellulaire par une série de centrifugations différentielles dans les conditions ci-après:

—une centrifugation 10 minutes à 7.500 g et ±4°C permet d'éliminen les cellules non broyées et les débris cellulaires. Le surnageant est ensuite soumis à une centrifugation de 45 minutes à 30.000 g et +4°C pour sédimenter la fraction membranaire brute.

—le culot 30.000 g est dispersé au moyen d'un homogénéiseur dans un volume de NaCl 0,15 M à froid puis soumis au même cycle de centrifugations à 7.500 g et 30.000 g que précédemment.

—le culot 30.000 g est repris cette fois dans un volume d'eau distillée et dispersé jusqu'à homogénéité parfaite.

—la suspension dans l'eau distillée est encore une fois soumise à un cycle de centrifugations à 7.500 g puis 30.000 g et le culot est repris cette fois dans 1/4 du volume initial d'eau distillée. La suspension est centrifugée 10 minutes à 7.500 g et le surnageant contenant les protéoglycanes bruts est conservé.

## Exemple 2

*Préparation de protéoglycanes purifiés*

—Au surnageant précédent obtenu à l'exemple 1 contenant les protéoglycanes bruts, on ajoute 1 ml d'hydroxyde de sodium (10 N) pour 100 ml de surnageant afin d'obtenir une molarité finale de 0,1 M en NaOH.

—On laisse incuber à 25°C pendant 24 heures avec une agitation modérée afin d'extraire par hydrolyse ménagée la fraction soluble des protéoglycanes.

—L'hydrolysat est ensuite neutralisé par une solution (3 N) d'acide chlorhydrique et le chlorure de sodium formé est éliminé par dialyse contre de l'eau distillée.

—Le résidu insoluble est éliminé après dialyse par une centrifugation de 45 minutes à 30.000 g et 4°C, le surnageant contenant les protéoglycanes solubles est conservé.

## Exemple 3

*Préparation de protéoglycanes purifiés.*

—Au surnageant obtenu à l'exemple 1 contenant les protéoglycanes bruts on ajoute 2 ml d'hypobromite de sodium (10 ml de brome + 60 ml d'hydroxyde de sodium concentré) pour 100 ml de surnageant.

—La suspension est maintenue pendant 30 minutes à température ambiante sous agitation.

—L'hypobromite en excès est alors éliminé par une dialyse de 24 heures contre de l'eau courante puis contre de l'eau distillée.

—Le résidu insoluble après dialyse est éliminé par centrifugation à 4°C pendant 45 minutes à 30.000 g et le surnageant contenant les protéoglycanes solubles est conservé.

## Exemple 4

Les protéoglycanes préparés à l'exemple 2 ne sont pas totalement détoxifiés. Ils contiennent encore de faibles quantités de lipopolysaccharides possédant un effet hyperthermisant. C'est pourquoi on traite les protéoglycanes de l'exemple 2 de la façon suivante:

—à 100 ml de surnageant dialysé contenant les protéoglycanes solubles, on ajoute 1 ml d'acide acétique glacial, puis on porte pendant 90 minutes à une température de 90°C.

Cette hydrolyse sépare quantitativement le lipide A du LPS et le refroidissement dans la glace entraîne sa précipitation.

Le précipité de Lipide A est éliminé par centrifugation 20 minutes à 30.000 g et 0°C.

Le surnageant est recueilli puis dialysé 24 heures contre de l'eau distillée pour éliminer l'acide acétique.

Le dialysat est stérilisé par filtration sur membrane 0,22 $\mu$ et le filtrat est conservé congelé ou bien lyophilisé.

Sa composition est la suivante:

| | |
|---|---|
| Hexoses | 37±5% |
| Protéines | 36,5±5% |
| Lipides | 15±3% |
| Hexosamines | 5±2% |
| ARN | <0,5% |
| ADN | ≪0,2% |
| LPS | <0,001% |

## Exemple 5

En opérant comme dans l'exemple 4 à partir des protéoglycanes purifiés de l'exemple 3, on obtient des protéoglycanes de la composition suivante:

| | |
|---|---|
| Hexoses | 29±5% |
| Protéines | 48±5% |
| Lipides | 15±3% |
| Hexosamines | 4±2% |
| ARN | <0,5% |

| ADN | ≪0,2% |
|---|---|
| LPS | <0,001% |

Bien entendu en opérant de la même façon à partir de Serratia marcescens et d'Escherichia coli on obtient les protéoglycanes purifiés et détoxifiés qui peuvent être utilisés dans les compositions décrites précédemment.

Les formulations de vaccins selon l'invention peuvent être conditionnées avec les supports et excipients connus dans ce domaine suivant leur mode d'application, les compositions qui ont été données précédemment sont de préférence des formes injectables.

Les vaccins selon la présente invention ont été testés pour leur activité immunogène et on a constate une activité sensiblement meilleure que celle des vaccins décrits au brevet français 2 253 499 due au remplacement des protéoglycanes membranaires par des protéoglycanes membranaires purifiés et détoxifiés. Mais l'aspect le plus avantageux des vaccins selon la présente invention est leur absence d'effet pyrogène.

Une étude de l'effet pyrogène a été conduite de la façon suivante:

On injecte par voie intraveineuse une dose de vaccin sous 1 ml d'eau par lapin de 2 à 2,5 kg et on observe la température rectale par sonde à thermocouple, les résultats observés sont donnés dans le Tableau I.

On constate que les vaccins selon la présente invention sont exempts d'effet pyrogène.

EFFET PYROGENE

| Lot | Δ°C | | | | | | | | | | | | | | |
|---|---|---|---|---|---|---|---|---|---|---|---|---|---|---|---|
| | 15' | 30' | 45' | 1h | 1h 15' | 1h 30' | 1h 45' | 2h | 2h 30' | 3 h | 3h 30' | 4h | 4h 30' | 5h | 5h 30' |
| GN 153 | 0,03 | 0 | 0 | −0,016 | −0,05 | −0,03 | −0,03 | −0,03 | −0,10 | −0,10 | 0 | −0,16 | −0,15 | −0,16 | −0,18 |
| GN 155 | +0,06 | +0,50 | +0,83 | +1,05 | +1,16 | +1,16 | +1,11 | +1,06 | +1,43 | +1.66 | +1,90 | +1,48 | +1,1 | +0,71 | +0,36 |
| GN 156 | +0,06 | −0,04 | −0,06 | −0,16 | −0,20 | −0,26 | −0,26 | −0,30 | −0,40 | −0.46 | −0,20 | −0,50 | −0,44 | −0,46 | −0,46 |

GN 153 — Vaccins antipyorrhéiques 2a, les protéoglycanes étant préparés par le procédé de l'exemple 4,

GN 156 — Mêmes vaccins, mais les protéoglycanes étant préparés par le procédé de l'exemple 5,

GN 155 — Mêmes vaccins que précédemment mais les protéoglycanes n'ayant subi qu'une délipidation totale au chloroforme.

**0013851**

## Revendications

1. Procédé de préparation de protéoglycanes membranaires bactériens purifiés, caractérisé en ce qu'il comporte au moins une étape de traitement des protéoglycanes bruts en milieu aqueux par une base ou un hypobromite suivi par l'élimination de l'excès de réactif et du résidu insoluble, les protéoglycanes purifiés se trouvant en solution aqueuse.

2. Procédé selon la revendication 1, caractérisé en ce que la base utilisée est un hydroxyde alcalin avec une molarité comprise entre 0,05 et 2 M et que la durée du traitement est de quelques heures à une température voisine de la température ambiante.

3. Procédé selon la revendication 1, caractérisé en ce que l'hypobromite utilisé est un hypobromite alcalin et que la durée du traitement est de quelques dizaines de minutes à la température ambiante.

4. Procédé selon l'une des revendications 1 à 3, caractérisé en ce que l'excès de réactif est éliminé par dialyse et le résidu insoluble est éliminé par centrifugation.

5. Procédé selon l'une des revendications 1 à 4, caractérisé en ce que le procédé de préparation comporte au moins une étape d'hydrolyse à l'acide acétique en milieu aqueux des protéoglycanes membranaires à une température comprise entre 70 et 100°C et élimination de la fraction insoluble.

6. Procédé selon la revendication 5, caractérisé en ce que l'hydrolyse acide est conduite à l'aide d'acide acétique glacial à 1% en volume.

7. Procédé selon l'une des revendications 1 à 6, caractérisé en ce que les protéoglycanes sont extraits de bactéries gram négatif.

8. Procédé selon la revendication 7, caractérisé en ce que les bactéries gram négatif sont choisies parmi:

Klebsiella pneumoniae
Serratia marcescens
Escherichia coli.

9. A titre d'adjuvants d'immunité pour vaccins, les protéoglycanes purifiés obtenus par la mise en oeuvre du procédé selon l'une des revendications 1 à 8.

10. A titre d'adjuvants d'immunité pour vaccins, les protéoglycanes solubles dans l'eau extraits de membranes bactériennes, obtenus par la mise en oeuvre du procédé selon l'une des revendications 1 à 8, ayant la composition suivante en poids:

| | |
|---|---|
| Hexoses | 24—42% |
| Protéines | 31—53% |
| Lipides | 12—18% |
| Hexosamines | 2—6% |
| ARN moins de | 0,5% |
| ADN moins de | 0,2% |
| LPS moins de | 0,001% |

11. A titre d'adjuvants selon la revendication 10, les protéoglycanes obtenus par la mise en oeuvre du procédé selon l'une des revendications 1 à 8 ayant la composition en poids:

| | |
|---|---|
| Hexoses | 37±5% |
| Protéines | 36,5±5% |
| Lipides | 15±3% |
| Hexosamines | 5±2% |
| ARN | <0,5% |
| ADN | ≪0,2% |
| LPS | <0,001% |

12. A titre d'adjuvants selon la revendication 10, les protéoglycanes obtenus par la mise en oeuvre du procédé selon l'une des revendications 1 à 8 ayant la composition suivante en poids:

12

**0 013 851**

| | |
|---|---|
| Hexoses | 29±5% |
| Protéines | 48±5% |
| Lipides | 15±3% |
| Hexosamines | 4±2% |
| ARN | <0,5% |
| ADN | ≪0,2% |
| LPS | <0,001% |

13. Vaccin à base de ribosome et/ou d'ARN bactérien à titre d'agent immunogène contenant à titre d'adjuvant d'immunité un adjuvant selon l'une des revendications 9 à 12.

14. Vaccin selon la revendication 13 ayant la composition suivante:

1) *Vaccins Broncho-ORL*

a) *Association Ribosomes-protéoglycanes*

| | |
|---|---|
| —ribosomes de Klebsiella pneumoniae | 3,5 $\mu$g |
| —ribosomes de Streptococcus pneumoniae | 3,0 $\mu$g |
| —ribosomes de Streptococcus pyogènes $A_{12}$ | 3,0 $\mu$g |
| —ribosomes d'Hemophilus influenzae | 0,5 $\mu$g |
| —Protéoglycanes de Klebsiella pneumoniae | 15,0 $\mu$g |

b) *Association ARN-ribosomal-protéoglycanes*

| | |
|---|---|
| —ARN ribosomal de Klebsiella pneumoniae | 2,45 $\mu$g |
| —ARN ribosomal de Streptococcus pneumoniae | 2,10 $\mu$g |
| —ARN ribosomal de Streptococcus pyogènes $A_{12}$ | 2,10 $\mu$g |
| —ARN ribosomal d'Hemophilus influenzae | 0,35 $\mu$g |
| —Protéoglycanes de Klebsiella pneumoniae | 10,50 $\mu$g |

2) *Vaccins anti-pyorrhéiques*

a) *Association Ribosomes-protéoglycanes*

| | |
|---|---|
| —ribosomes de Rothia dentocariosus | 1,2 $\mu$g |
| —ribosomes d'Actinomyces viscosus | 1,2 $\mu$g |
| —ribosomes de Streptococcus mutans | 0,8 $\mu$g |
| —ribosomes de Streptococcus salivarius | 2,0 $\mu$g |
| —ribosomes de Lactobacillus casei | 1,2 $\mu$g |
| —Protéoglycanes de Klebsiella pneumoniae | 9,6 $\mu$g |

b) *Association ARN-ribosomal Protéoglycanes*

| | |
|---|---|
| —ARN ribosomal de Rethia dentocariosus | 0,84 $\mu$g |
| —ARN ribosomal d'Actinomyces viscosus | 0,84 $\mu$g |

13

**0 013 851**

—ARN ribosomal de Streptococcus mutans     0,56 $\mu$g

—ARN ribosomal de Streptococcus salivarius     1,40 $\mu$g

—ARN ribosomal de Lactobacillus casei     0,84 $\mu$g

—Protéoglycanes de Klebsiella pneumoniae     6,72 $\mu$g

3) *Vaccins dermatologique, anti-acnéique*

    a) *Association ribosomes—Protéoglycanes*

    —ribosomes de Corynebacterium acnes     2 $\mu$g

    —ribosomes de Corynebacterium parvum     2 $\mu$g

    —ribosomes de Streptococcus pyogènes     2 $\mu$g

    —ribosomes de Staphylococcus epidermidis     2 $\mu$g

    —Protéoglycanes de Serratia marcescens     12 $\mu$g

    b) *Association ARN ribosomal—Protéoglycanes*

    —*ARN ribosomal de Corynebacterium acnes*     1,4 $\mu$g

    —ARN ribosomal de Corynebacterium parvum     1,4 $\mu$g

    —ARN ribosomal de Streptococcus pyogènes     1,4 $\mu$g

    —ARN ribosomal de Staphylococcus epidermidis     1,4 $\mu$g

    —Protéoglycanes de Serratia marcescens     8,4 $\mu$g

4) *Vaccin gynécologique*

    a) *Association ribosomes-protéoglycanes*

    —ribosomes d'Escherichia coli     2 $\mu$g

    —ribosomes de Streptococcus faecalis D     2 $\mu$g

    —ribosomes de Streptococcus H     2 $\mu$g

    —ribosomes de Staphylococcus epidermidis     2 $\mu$g

    —ribosomes de Candida albicans     2 $\mu$g

    —Protéoglycanes d'Escherichia coli     15 $\mu$g

    b) *Association ARN ribosomal Protéoglycanes*

    —ARN ribosomal d'Escherichia coli     1,4 $\mu$g

    —ARN ribosomal de Streptococcus faecalis D     1,4 $\mu$g

    —ARN ribosomal de Streptococcus H     1,4 $\mu$g

    —ARN ribosomal de Staphylococcus epidermidis     1,4 $\mu$g

    —ARN ribosomal de Candida albicans     1,4 $\mu$g

    —Protéoglycanes d'Escherichia coli     10,5 $\mu$g

**0013851**

5) *Vaccin intestinal, anti-typhique*

    a) *Association Ribosomes-Protéoglycanes*

| | |
|---|---|
| —ribosomes de Bacterium coli | 2 $\mu$g |
| —ribosomes de Salmonella paratyphi A | 2 $\mu$g |
| —ribosomes de Salmonella paratyphi B | 2 $\mu$g |
| —ribosomes de Shigella dydenteria | 2 $\mu$g |
| —ribosomes d'Enterococcus | 2 $\mu$g |
| —Protéoglycanes de Serratia marcescens | 15 $\mu$g |

    b) *Association ARN ribosomal—Protéoglycanes*

| | |
|---|---|
| —ARN ribosomal de Bacterium coli | 1,4 $\mu$g |
| —ARN ribosomal de Salmonella paratyphi A | 1,4 $\mu$g |
| —ARN ribosomal de Salmonella paratyphi B | 1,4 $\mu$g |
| —ARN ribosomal de Shigella dysenteriae | 1,4 $\mu$g |
| —ARN ribosomal d'Enterococcus | 1,4 $\mu$g |
| —Protéoglycanes de Serratia marcescens | 10,5 $\mu$g |

## Claims

1. A process for the preparation of purified bacterial membranal proteoglycans characterized in that it comprises at least one step in which crude proteoglycans are treated in an aqueous medium with a reactant selected from the group consisting of bases and hypobromites, followed by removal of the excess reactant and the insoluble residue, the purified proteoglycans being present in aqueous solution.

2. A process as set forth in claim 1, characterized in that the base used is an alkali hydroxide having a molarity of from 0.05 to 2 m and the treatment time is a few hours at a temperture of about ambient temperature.

3. A process as set forth in claim 1, characterized in that the hypobromite used is an alkali hypobromite and the treatment time is a few tens of minutes at ambient temperature.

4. A process as set forth in any one of claims 1 to 3, characterized in that the excess reactant is eliminated by dialysis and the insoluble residue is eliminated by centrifuging.

5. A process as claimed in any one of claims 1 to 4, which further comprises at least one step in which the membranal proteoglycans are hydrolysed with acetic acid in an aqueous medium at a temperature of from 70 to 100°C and the thus formed insoluble fraction is eliminated.

6. A process as claimed in claim 5, characterized in that the acid hydrolysis step is carried out with 1% by volume glacial acetic acid.

7. A process as claimed in any one of claims 1 to 6, characterized in that the proteoglycans are extracted from a gram-negative bacteria.

8. A process as claimed in claim 7, characterized in that the proteoglycans are extracted from gram-negative bacteria selected from the group consisting of *Klebsiella, pneumoniae, Serratia marcescens* and *Escherichia coli.*

9. As immunity adjuvant for vaccines, the purified proteoglycans obtained by a process according to any one of claims 1 to 8.

10. As immunity adjuvant for vaccines the water-soluble proteoglycans extracted from bacterial membranes obtained by the process according to any one of claims 1 to 8 having the following composition by weight:

| | |
|---|---|
| hexoses | 24—42% |
| proteins | 31—53% |
| lipids | 12—18% |

15

**0 013 851**

| | |
|---|---|
| hexosamines | 2—6% |
| RNA | <0.5% |
| DNA | <0.2% |
| LPS | <0.001% |

11. As adjuvant according to claim 10, the proteoglycans obtained by the process according to any one of claims 1 to 8 having the following composition by weight:

| | |
|---|---|
| hexoses | $37\pm5\%$ |
| proteins | $36.5\pm5\%$ |
| lipids | $15\pm3\%$ |
| hexosamines | $5\pm2\%$ |
| RNA | <0.5% |
| DNA | ≪0.2% |
| LPS | <0.001% |

12. As adjuvant according to claim 10, the proteoglycans obtained by the process according to any one of claims 1 to 8 having the following composition by weight:

| | |
|---|---|
| hexoses | $29\pm5\%$ |
| proteins | $48\pm5\%$ |
| lipids | $15\pm3\%$ |
| hexosamines | $4\pm2\%$ |
| RNA | <0.5% |
| DNA | ≪0.2% |
| LPS | <0.001% |

13. A vaccine based on bacterial ribosome and/or RAN as immunogenic agent containing as immunity adjuvant an adjuvant according to any one of claims 9 to 12.

14. A vaccine as set forth in claim 13 having the following composition:

1) Broncho ORL vaccine

a) association of ribosomes-proteoglycans

| | |
|---|---|
| —ribosomes of *Klebsiella pneumoniae* | 3.5 $\mu$g |
| —ribosomes of *Streptococcus pneumoniae* | 3.0 $\mu$g |
| —ribosomes of *Streptococcus pyogenes* $A_{12}$ | 3.0 $\mu$g |
| —ribosomes of *Hemophilus influenzae* | 0.5 $\mu$g |
| —proteoglycans of *Klebsiella pneumoniae* | 15.0 $\mu$g |

b) association ribosomal RNA-proteoglycans

| | |
|---|---|
| —ribosomal RNA of *Klebsiella pneumoniae* | 2.45 $\mu$g |
| —ribosomal RNA of *Streptococcus pneumoniae* | 2.10 $\mu$g |

16

**0 013 851**

—ribosomal RNA of *Streptococcus pyogenes* $A_{12}$ — 2.10 μg

—ribosomal RNA of *Hemophilus influenzae* — 0.35 μg

—proteoglycans of *Klebsiella pneumoniae* — 10.50 μg

2) Anti-pyorrheic vaccine

  a) association ribosomes-proteoglycans

—ribosomes of *Rothia dentocariosus* — 1.2 μg

—ribosomes of *Actinomyces viscosus* — 1.2 μg

—ribosomes of *Streptococcus mutans* — 0.8 μg

—ribosomes of *Streptococcus salivarius* — 2.0 μg

—ribosomes of *Lactobacillus casei* — 1.2 μg

—proteoglycans of *Klebsiella pneumoniae* — 9.6 μg

  b) association ribosomal RNA-proteoglycans

—ribosomal RNA of *Rothia dentocariosus* — 0.84 μg

—ribosomal RNA of *Actinomyces viscosus* — 0.84 μg

—ribosomal RNA of *Streptococcus mutans* — 0.56 μg

—ribosomal RNA of Streptococcus salivarius — 1.40 μg

—ribosomal RNA of *Lactobacillus casei* — 0.84 μg

—proteoglycans of *Klebsiella pneumoniae* — 6.72 μg

3) Dermatological vaccine anti-acneic

  a) association ribosomes-proteoglycans

—ribosomes of *Corynebacterium acnes* — 2 μg

—ribosomes of *Corynebacterium parvum* — 2 μg

—ribosomes of *Streptococcus pyogenes* — 2 μg

—ribosomes of *Staphylococcus epidermidis* — 2 μg

—proteoglycans of *Serratia marcescens* — 12 μg

  b) association ribosomal RNA-proteoglycans

—ribosomal RNA of *Corynebacterium acnes* — 1.4 μg

—ribosomal RNA of *Corynebacterium parvum* — 1.4 μg

—ribosomal RNA of *Streptococcus pyogenes* — 1.4 μg

—ribosomal RNA of *Staphylococcus epidermidis* — 1.4 μg

—proteoglycans of *Serratia marcescens* — 8.4 μg

17

4) Gynecologic vaccine

   a) association of ribosomes-proteoglycans

| | |
|---|---|
| —ribosomes of *Escherichia coli* | 2 μg |
| —ribosomes of *Streptococcus faecalis* | 2 μg |
| —ribosomes of *Streptococcus* H | 2 μg |
| —ribosomes of *Staphylococcus epidermidis* | 2 μg |
| —ribosomes of *Candida albicans* | 2 μg |
| —proteoglycans of *Escherichia coli* | 15 μg |

   b) association of ribosomal RNA-proteoglycans

| | |
|---|---|
| —ribosomal RNA of *Escherichia coli* | 1.4 μg |
| —ribosomal RNA of *Streptococcus faecalis* D | 1.4 μg |
| —ribosomal RNA of *Streptococcus* H | 1.4 μg |
| —ribosomal RNA of *Staphylococcus epidermidis* | 1.4 μg |
| —ribosomal RNA of *Candida albicans* | 1.4 μg |
| —proteoglycans of *Escherichia coli* | 10.5 μg |

5) Intestinal vaccine—anti-typhous

   a) association ribosomes-proteoglycans

| | |
|---|---|
| —ribosomes of *Bacterium coli* | 2 μg |
| —ribosomes of *Salmonella paratyphi* A | 2 μg |
| —ribosomes of *Salmonella paratyphi* B | 2 μg |
| —ribosomes of *Shigella dysenteria* | 2 μg |
| —ribosomes of *Enterococcus* | 2 μg |
| —proteoglycans of *Serratia marcescens* | 15 μg |

   b) association ribosomal RNA-proteoglycans

| | |
|---|---|
| —ribosomal RNA of *Bacterium coli* | 1.4 μg |
| —ribosomal RNA of *Salmonella paratyphi* A | 1.4 μg |
| —ribosomal RNA of *Salmonella paratyphi* B | 1.4 μg |
| —ribosomal RNA of *Shigella dysenteriae* | 1.4 μg |
| —ribosomal RNA of *Enterococcus* | 1.4 μg |
| —proteoglycans of *Serratia marcescens* | 10.5 μg |

**Patentansprüche**

1. Verfahren zur Herstellung von gereinigten Proteoglykanen aus Bakterienmembranen, dadurch gekennzeichnet, daß man wenigstens eine Stufe der Behandlung der rohen Proteoglykane in

**0 013 851**

wässerigem Milieu durch eine Base oder ein Hypobromit durchführt, worauf der Reaktantenüberschuß und der unlösliche Rückstand entfernt werden und die gereinigten Proteoglykane in wässeriger Lösung vorhanden sind.

2. Verfahren nach Anspruch 1, dadurch gekennzeichnet, daß die verwendete Base ein Alkalihydroxid mit einer Molarität zwischen 0,05 und 2 M ist und daß die Behandlungsdauer mehrere Stunden bei einer Temperatur nahe der Umgebungstemperatur beträgt.

3. Verfahren nach Anspruch 1, dadurch gekennzeichnet, daß das verwendete Hypobromit ein Alkalihypobromit ist und daß die Behandlungsdauer etliche 10 min bei Umgebungstemperatur beträgt.

4. Verfahren nach einem der Ansprüche 1 bis 3, dadurch gekennzeichnet, daß der Reaktantenüberschuß durch Dialyse und der unlösliche Rückstand durch Zentrifugieren entfernt werden.

5. Verfahren nach einem der Ansprüche 1 bis 4, dadurch gekennzeichnet, daß das Herstellungsverfahrens wenigstens eine Hydrolysestufe mit Essigsäure in wässerigem Milieu der Proteoglykane von Membranen bei einer Temperatur zwischen 70 und 100°C und die Entfernung der unlöslichen Fraktion umfaßt.

6. Verfahren nach Anspruch 5, dadurch gekennzeichnet, daß die saure Hydrolyse mit 1 Vol.% Eisessig durchgeführt wird.

7. Verfahren nach einem der Ansprüche 1 bis 6, dadurch gekennzeichnet, daß die Proteoglykane Extrakte Gram-negativer Bakterien sind.

8. Verfahren nach Anspruch 7, dadurch gekennzeichnet, daß die Gram-negativen Bakterien unter
Klebsiella pneumoniae,
Serratia marcescens und
Escherichia coli
gewählt sind.

9. Die durch das Verfahren nach einem der Ansprüche 1 bis 8 erhaltenen gereinigten Proteoglykane als Immunitäts-adjuvantien für Vakzinen.

10. Die durch das Verfahren nach einem der Ansprüche 1 bis 8 erhaltenen, wasserlöslichen, aus Bakterienmembranen extrahierten Proteoglykane als Immunitätsadjuvantien für Vakzinen mit folgender Zusammensetzung in Gew.%:

| | |
|---|---|
| Hexosen | 24—42% |
| Proteine | 31—53% |
| Lipide | 12—18% |
| Hexosamine | 2—6% |
| RNS weniger als | 0,5% |
| DNS weniger als | 0,2% |
| LPS weniger als | 0,001% |

11. Die durch das Verfahren nach einem der Ansprüche 1 bis 8 erhaltenen Proteoglykane als Adjuvantien gemäß Anspruch 10 mit folgender Zusammensetzung in Gew.%:

| | |
|---|---|
| Hexosen | $37\pm5\%$ |
| Proteine | $36,5\pm5\%$ |
| Lipide | $15\pm3\%$ |
| Hexosamine | $5\pm2\%$ |
| RNS | $<0,5\%$ |
| DNS | $\ll0,02\%$ |
| LPS | $<0,001\%$ |

12. Die durch das Verfahren nach einem der Ansprüche 1 bis 8 erhaltenen Proteoglykane als Adjuvantien gemäß Anspruch 10 mit folgender Zusammensetzung in Gew.%:

| | |
|---|---|
| Hexosen | $29\pm5\%$ |

19

**0 013 851**

| | |
|---|---|
| Proteine | 48±5% |
| Lipide | 15±3% |
| Hexosamine | 4±2% |
| RNS | <0,5% |
| DNS | ≪0,2% |
| LPS | <0,001% |

13. Vakzine auf Basis von Ribosom und/oder bakterieller RNS als immunologisches Mittel, das als Immunitätsadjuvans ein Adjuvans gemäß einem der Ansprüche 9 bis 12 enthält.

14. Vakzine nach Anspruch 13 mit folgender Zusammensetzung:

1) Broncho-ORL-Vakzinen

a) *Assoziation Ribosomen—Proteoglykane*

| | |
|---|---|
| Ribosomen von Klebsiella pneumoniae | 3,5 $\mu$g |
| Ribosomen von Streptococcus pneumoniae | 3,0 $\mu$g |
| Ribosomen von Streptococcus pyogenes $A_{12}$ | 3,0 $\mu$g |
| Ribosomen von Haemophilus influenzae | 0,5 $\mu$g |
| Proteoglykane von Klebsiella pneumoniae | 15,0 $\mu$g |

b) *Assoziation ribosomische RNS—Proteoglykane*

| | |
|---|---|
| ribosomische RNS von Klebsiella pneumoniae | 2,45 $\mu$g |
| ribosomische RNS von Streptococcus pneumoniae | 2,10 $\mu$g |
| ribosomische RNS von Streptococcus pyogenes $A_{12}$ | 2,10 $\mu$g |
| ribosomische RNS von Haemophilus influenzae | 0,35 $\mu$g |
| Proteoglykane von Klebsiella pneumoniae | 10,50 $\mu$g |

2) *Antipyorrhöe-Vakzinen*

a) *Assoziation Ribosomen—Proteoglykane*

| | |
|---|---|
| Ribosomen von Rothia dentocariosus | 1,2 $\mu$g |
| Ribosomen von Actinomyces viscosus | 1,2 $\mu$g |
| Ribosomen von Streptococcus mutans | 0,8 $\mu$g |
| Ribosomen von Streptococcus salivarius | 2,0 $\mu$g |
| Ribosomen von Lactobacillus casei | 1,2 $\mu$g |
| Proteoglykane von Klebsiella pneumoniae | 9,6 $\mu$g |

b) *Assoziation ribosomische RNS—Proteoglykane*

| | |
|---|---|
| ribosomische RNS von Rethia dentocariosus | 0,84 $\mu$g |
| ribosomische RNS von Actinomyces viscosus | 0,84 $\mu$g |
| ribosomische RNS von Streptococcus mutans | 0,56 $\mu$g |

20

ribosomische RNS von Streptococcus salivarius | 1,40 µg

ribosomische RNS von Lactobacillus casei | 0,84 µg

Proteoglykane von Klebsiella pneumoniae | 6,72 µg

3) *Dermatologische Antiakne-Vakzinen*

a) *Assoziation Ribosomen—Proteoglykane*

Ribosomen von Corynebacterium acnes | 2 µg

Ribosomen von Corynebacterium parvum | 2 µg

Ribosomen von Streptococcus pyogenes | 2 µg

Ribosomen von Staphylococcus epidermidis | 2 µg

Proteoglykane von Serratia marcescens | 12 µg

b) *Assoziation ribosomische RNS—Proteoglykane*

ribosomische RNS von Corynebacterium acnes | 1,4 µg

ribosomische RNS von Corynebacterium parvum | 1,4 µg

ribosomische RNS von Streptococcus pyogenes | 1,4 µg

ribosomische RNS von Staphylococcus epidermidis | 1,4 µg

Proteoglykane von Serratia marcescens | 8,4 µg

4) *Gynäkologische Vakzine*

a) *Assoziation Ribosomen—Proteoglykane*

Ribosomen von Escherichia coli | 2 µg

Ribosomen von Streptococcus faecalis D | 2 µg

Ribosomen von Streptococcus H | 2 µg

Ribosomen von Staphylococcus epidermidis | 2 µg

Ribosomen von Candida albicans | 2 µg

Proteoglykane von Escherichia coli | 15 µg

b) *Assoziation ribosomische RNS—Proteoglykane*

ribosomische RNS von Escherichia coli | 1,4 µg

ribosomische RNS von Streptococcus faecalis D | 1,4 µg

ribosomische RNS von Streptococcus H | 1,4 µg

ribosomische RNS von Staphylococcus epidermidis | 1,4 µg

ribosomische RNS von Candida albicans | 1,4 µg

Proteoglykane von Escherichia coli | 10,5 µg

**0 013 851**

5) *Antityphus—Intestinalvakzine*

    a) *Assoziation Ribosomen—Proteoglykane*

| | |
|---|---|
| Ribosomen von Bacterium coli | 2 µg |
| Ribosomen von Salmonella paratyphi A | 2 µg |
| Ribosomen von Salmonella paratyphi B | 2 µg |
| Ribosomen von Shigella dydenteria | 2 µg |
| Ribosomen von Enterococcus | 2 µg |
| Proteoglykane von Serratia marcescens | 15 µg |

    b) *Assoziation ribosomische RNS—Proteoglykane*

| | |
|---|---|
| ribosomische RNS von Bacterium coli | 1,4 µg |
| ribosomische RNS von Salmonella paratyphi A | 1,4 µg |
| ribosomische RNS von Salmonella paratyphi B | 1,4 µg |
| ribosomische RNS von Shigella dysenteriae | 1,4 µg |
| ribosomische RNS von Enterococcus | 1,4 µg |
| Proteoglykane von Serratia marcescens | 10,5 µg |